# EUROPEAN PATENT APPLICATION

(11) **EP 4 410 225 A1**
(43) Date of publication of application: **07.08.2024**
(21) Application number: 24155184.5
(22) Date of filing: 01.02.2024
(51) Int. Cl.: A61B 17/22, A61M 25/10

(54) **VASCULAR OCCLUSION CATHETER FOR PARTIAL OCCLUSION OR FULL OCCLUSION**

(30) Priority: 03.02.2023 US 202363443064 P
(71) Applicant: Prytime Medical Devices, Inc., Boerne, TX 78006 (US)
(72) Inventor: FRANKLIN, Curtis J., Colorado, 80228 (US); KRUMMENACHER, Todd J., Colorado, 80228 (US)
(74) Representative: Walther Bayer Faber Patentanwälte PartGmbB

(57) **Abstract**

A vascular occlusion catheter may be employed for at least partial occlusion of a target vessel. The vascular occlusion catheter system includes a proximal catheter shaft having at least one internal lumen, and a distal catheter shaft terminating in an atraumatic tip. An occlusion balloon is sealingly mounted to the proximal and distal catheter shafts. A central catheter shaft extends through the proximal catheter shaft, the occlusion balloon and into the distal catheter shaft. The proximal catheter shaft is secured to the central catheter shaft on a proximal side of the occlusion balloon and the distal catheter shaft is secured to the central catheter shaft on a distal side of the occlusion balloon. The occlusion balloon, the proximal catheter shaft and the distal catheter shaft have a greatest outer diameter of less than seven French (7 Fr) in an uninflated condition.

## Description

### CROSS-REFERENCE TO RELATED APPLICATION

This application claims priority from U.S. Provisional Patent Application No. 63/443,064, filed February 3, 2023, titled "Vascular Occlusion Catheter", the entire contents of which are incorporated by reference herein.

### BACKGROUND OF THE DISCLOSURE

This disclosure relates to vascular occlusion catheters and, more particularly, to vascular occlusion catheters capable of performing both partial and full vascular occlusion.

Vascular occlusion may be indicated in either the venous system and/or the arterial system. Endoarterial occlusion, such as, resuscitative endovascular balloon occlusion of the aorta ("REBOA"), is a procedure in which a blood vessel is temporarily at least partially occluded in order to restrict blood flow upstream or downstream of the occlusion site for purposes of a vascular procedure or repair. Partial occlusion of the aorta is beneficial to mitigate the risk of ischemia below (downstream of) the occlusion site to limit the lack of blood flow to organs and tissue below the occlusion site. That is, partial perfusion past the occlusion balloon can provide the benefits of focusing or directing a majority of blood flow to the brain, heart and lungs or other upstream portions of the patient, while also potentially increasing the amount of time the occlusion balloon can be implanted in the patient by providing at least partial blood flow to the patient's organs downstream of the occlusion member, such as to the patient's liver, digestive tract, kidneys and legs.

Traditionally, an inserter or introducer cannula/sheath is utilized for introducing an occlusion catheter into a patient and remains in the access site for retraction and/or exchange of the catheter upon completion of the REBOA procedure. The profile of the catheter, including that of the occlusion balloon, directly corresponds to a profile of the sheath, and, in turn, the size of the access site incision. That is, a greatest outer diameter of the catheter should be small enough for insertion into the introducer sheath. Any increase in the size or diameter of the catheter shaft results in an increase in size or counterpart dimension of the introducer sheath, and, accordingly, an increase in the access site incision in the patient's body. A seven French (7 Fr) or smaller introducer sheath typically results in the access site (through the patient's skin and into the target vessel) being re-closable by holding manual pressure for a period of time, such as twenty to thirty minutes (20-30 min). If the introducer sheath has an inner diameter greater than seven French (7 Fr), surgical repair of the access site may be required, thereby further complicating the procedure.

One approach to assembling a catheter that will successfully pass through the introducer sheath, *e.g*., a 7 Fr introducer sheath, is to assemble the uninflated/folded occlusion balloon to the remainder of the catheter in a manner where a requisite amount of the axial slack is removed from the occlusion balloon, *i.e.*, attaching the uninflated occlusion balloon to the catheter in a compressed and folded, at least partially taut manner. Otherwise, additional slack in the uninflated/folded occlusion balloon may thicken portions of the occlusion balloon's radial dimension to greater than 7 Fr. Naturally, therefore, when the occlusion balloon is subsequently inflated during use, whether partially or fully, the occlusion balloon applies a tensile force on both opposing neck portions of the balloon while the central diameter of the occlusion balloon increases. In turn, the neck portions of the occlusion balloon transfer the tensile forces onto other components of the catheter.

After completion of the REBOA procedure, one prospective anomaly may present itself if the occlusion balloon is not fully deflated before attempting to withdraw the catheter from the introducer sheath. When the user pulls on the catheter to remove it, the balloon may resist entering or sliding through the introducer sheath due to the residual fluid within the balloon. Further pulling may exacerbate the issue by plastically deforming components of the catheter under the tensile loads applied thereon. Once components of the catheter are plastically deformed, even if the occlusion balloon is subsequently fully deflated, the balloon may not return to the same folded, at least partially taut state exhibited prior to use, but rather exhibit increased slack. The resulting, increased axial slack in the occlusion balloon may, in turn, result in an enlarged radial profile of the catheter after use, complicating the withdrawal of the catheter from the introducer sheath.

It would, therefore, be desirable to design, develop and implement an occlusion balloon catheter configured to exhibit substantially the same radial profile before and after use for successful insertion and withdrawal from the accompanying introducer sheath.

### BRIEF SUMMARY OF THE DISCLOSURE

Briefly stated, one aspect of the present disclosure is directed to a vascular occlusion catheter employed for at least partial occlusion of a target vessel. The vascular occlusion catheter system includes a proximal catheter shaft having at least one internal lumen, and a distal catheter shaft terminating in an atraumatic tip. An occlusion balloon is sealingly mounted to the proximal and distal catheter shafts. A central catheter shaft extends through the proximal catheter shaft, the occlusion balloon and into the distal catheter shaft. The proximal catheter shaft is secured to the central catheter shaft on a proximal side of the occlusion balloon and the distal catheter shaft is secured to the central catheter shaft on a distal side of the occlusion balloon. The occlusion balloon, the proximal catheter shaft and the distal catheter shaft have a greatest outer diameter of less than seven French (7 Fr) in an uninflated condition.

In one configuration, a proximal neck portion of the occlusion balloon is thermally bonded to the proximal catheter shaft and a distal neck portion of the occlusion balloon is thermally bonded to the distal catheter shaft. In one configuration, each of the proximal and distal neck portions transitions into a generally conical portion. In one configuration, at least one of (i) the proximal neck portion and the corresponding generally conical portion define a radiused transition therebetween, or (ii) the distal neck portion and the corresponding generally conical portion define a radiused transition therebetween.

In any one of the previous configurations, the central catheter shaft may extend through a first internal lumen of the at least one internal lumen of the proximal catheter shaft and the at least one internal lumen further includes a second internal lumen. In one configuration, the catheter further includes a proximal window formed in a sidewall of the proximal catheter shaft and extending into the second internal lumen, and a proximal sensor is positioned within the proximal window. In any one of the previous configurations, the second internal lumen may distally terminate proximal to the occlusion balloon. In one configuration, the proximal catheter shaft is thermally bonded to the central catheter shaft distal to the second internal lumen.

In any one of the previous configurations, the proximal catheter shaft may be thermally bonded to the central catheter shaft. In one configuration, at least a segment of a proximal neck portion of the occlusion balloon is thermally bonded to the proximal catheter shaft, the bond between the proximal neck portion and the proximal catheter shaft at least partially radially overlapping with the bond between the proximal catheter shaft and the central catheter shaft, along an axial axis of the catheter, thereby forming a proximal three-component-attachment. In one configuration, the proximal three-component-attachment is between approximately 0.5 mm and approximately 15 mm in axial length. In any one of the previous configurations, the at least one internal lumen may include a proximal shaft lumen which distally transitions into at least one internal transition lumen axially extending entirely through the thermal bond, thereby fluidly connecting the proximal shaft lumen with an interior of the occlusion balloon.

In any one of the previous configurations, the securement between the proximal catheter shaft and the central catheter shaft jackets an entire circumferential periphery of the central catheter shaft.

In any one of the previous configurations, the distal catheter shaft is thermally bonded to the central catheter shaft. In one configuration, at least a segment of a distal neck portion of the occlusion balloon is thermally bonded to the distal catheter shaft, the bond between the distal neck portion and the distal catheter shaft at least partially radially overlapping with the bond between the distal catheter shaft and the central catheter shaft, along an axial axis of the catheter, thereby forming a distal three-component-attachment. In one configuration, the proximal three-component-attachment is between approximately 0.5 mm and approximately 20 mm in axial length.

In any one of the previous configurations, the central catheter shaft may extend into a distal internal lumen of the distal catheter shaft, a distal window being formed in a sidewall of the distal catheter shaft and extending into the distal internal lumen, and the catheter further includes a distal sensor positioned within the distal window.

In any one of the previous configurations, the distal catheter shaft may define a distal internal lumen terminating in an open distal port proximate the atraumatic tip, the central catheter shaft distally terminating within the distal internal lumen and being in fluid communication with the open distal port.

In any one of the previous configurations, the occlusion balloon may define a proximal neck portion, a distal neck portion and an intervening central body portion, wherein at least one of the proximal neck portion or the distal neck portion is constructed of a material having a lower durometer than the central body portion. In one configuration, the at least one of the proximal neck portion or the distal neck portion defines a tensile modulus that is between approximately 0.5% and approximately 80% of a tensile modulus of the central body portion.

### BRIEF DESCRIPTION OF THE SEVERAL VIEWS OF THE DRAWINGS

The following description of embodiments of the disclosure will be better understood when read in conjunction with the appended drawings. It should be understood, however, that the disclosure is not limited to the precise arrangements and instrumentalities shown. In the drawings:
Fig. 1 is a partial, side elevational view of an occlusion catheter according to a first embodiment of the present disclosure;
Fig. 2 is an enlarged, partial cross-sectional view of a proximal section of the occlusion catheter of Fig. 1, taken along sectional line 2-2;
Fig. 3 is an enlarged, partial cross-sectional view of a proximal section of the occlusion catheter of Fig. 1, taken along a sectional line perpendicular to sectional line 2-2;
Fig. 4 is an enlarged, partial perspective view of a proximal neck portion of the occlusion balloon of the occlusion catheter of Fig. 1;
Fig. 5 is an enlarged, partial cross-sectional view of a distal section of the occlusion catheter of Fig. 1, taken along sectional line 2-2;
Fig. 6 is an enlarged, partial cross-sectional view of an alternative configuration of the distal section of the occlusion catheter of Fig. 1, taken along sectional line 2-2;
Fig. 7 is an enlarged, side elevational view of the occlusion balloon of the occlusion catheter of Fig. 1;
Fig. 8 is an enlarged, partial cross-sectional view of the distal section of the occlusion catheter of Fig. 1, taken along sectional line 2-2;
Fig. 9 is an enlarged, partial perspective view of an alternative configuration of the proximal portion of the occlusion catheter of Fig. 1;
Fig. 10 is an enlarged, partial perspective view of a proximal neck portion of an occlusion catheter according to a second embodiment of the present disclosure with the occlusion balloon in an uninflated or deflated state;
Fig. 11 is an enlarged, partial cross-sectional view of a proximal section of the occlusion catheter of Fig. 10, taken along a sectional line perpendicular to sectional line 10-10 of Fig. 10;
Fig. 12 is an enlarged, partial perspective view of the proximal neck portion of the occlusion catheter of Fig. 10, with the occlusion balloon in an inflated state; and
Fig. 13 is an enlarged, partial cross-sectional view of the proximal section of the occlusion catheter of Fig. 12, taken along a sectional line perpendicular to sectional line 12-12 of Fig. 12.

### DETAILED DESCRIPTION OF THE DISCLOSURE

Certain terminology is used in the following description for convenience only and is not limiting. The words "lower," "bottom," "upper" and "top" designate directions in the drawings to which reference is made. The words "inwardly," "outwardly," "upwardly" and "downwardly" refer to directions toward and away from, respectively, the geometric center of the occlusion balloon catheter, and designated parts thereof, in accordance with the present disclosure. In describing the occlusion balloon catheter, the term proximal is used in relation to the end of the catheter closer to the user and the term distal is used in relation to the end of the catheter further from the user. Unless specifically set forth herein, the terms "a," "an" and "the" are not limited to one element, but instead should be read as meaning "at least one." The terminology includes the words noted above, derivatives thereof and words of similar import.

It should also be understood that the terms "about," "approximately," "generally," "substantially" and like terms, used herein when referring to a dimension or characteristic of a component of the disclosure, indicate that the described dimension/characteristic is not a strict boundary or parameter and does not exclude minor variations therefrom that are functionally similar. At a minimum, such references that include a numerical parameter would include variations that, using mathematical and industrial principles accepted in the art (e.g., rounding, measurement or other systematic errors, manufacturing tolerances, etc.), would not vary the least significant digit.

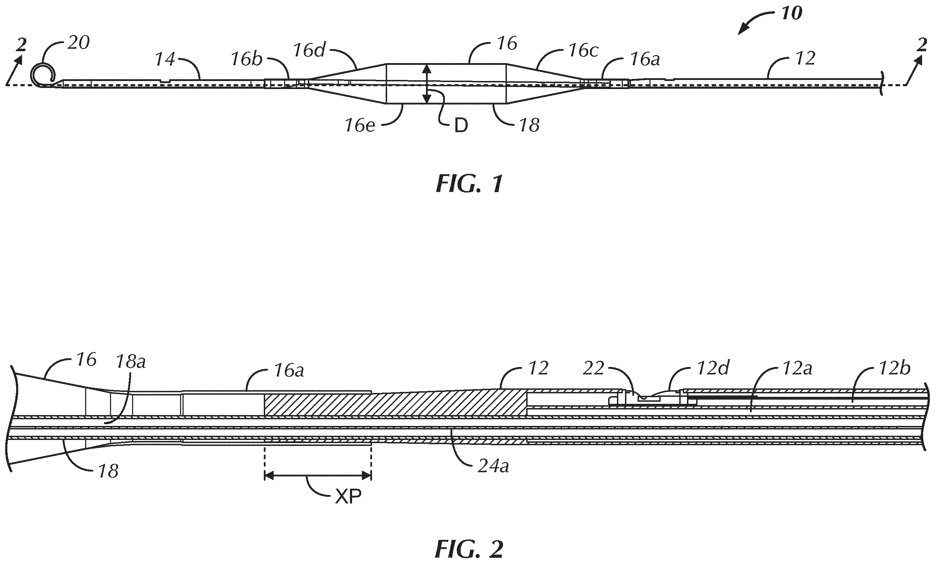

Referring to the drawings in detail, wherein like numerals indicate like elements throughout, there is shown in Figs. 1-9 an occlusion catheter, generally designated 10, in accordance with a first embodiment of the present disclosure. As shown, the catheter 10 includes a proximal catheter shaft 12, a distal catheter shaft 14 and an occlusion balloon 16 mounted to the proximal and distal catheter shafts 12, 14. For example, a proximal neck portion 16a of the occlusion balloon 16 may be sealingly mounted/anchored (in a manner understood, e.g., bonding, welding, a combination thereof or the like) to the proximal catheter shaft 12. Similarly, a distal neck portion 16b of the occlusion balloon 16 may be sealingly mounted/anchored (in a manner understood, e.g., bonding, welding, a combination thereof or the like) to the distal catheter shaft 14. The distal catheter shaft 14 distally terminates in an atraumatic tip or a P-tip 20. In one configuration, the proximal and distal catheter shafts 12, 14 may be constructed of medical grade, biocompatible polymers, such as, for example, without limitation, silicone, nylon, polyurethane, PETE, latex, thermoplastic elastomers, polyether block amides (e.g., PEBAX, Arkema, Paris, France), a combination thereof, or the like.

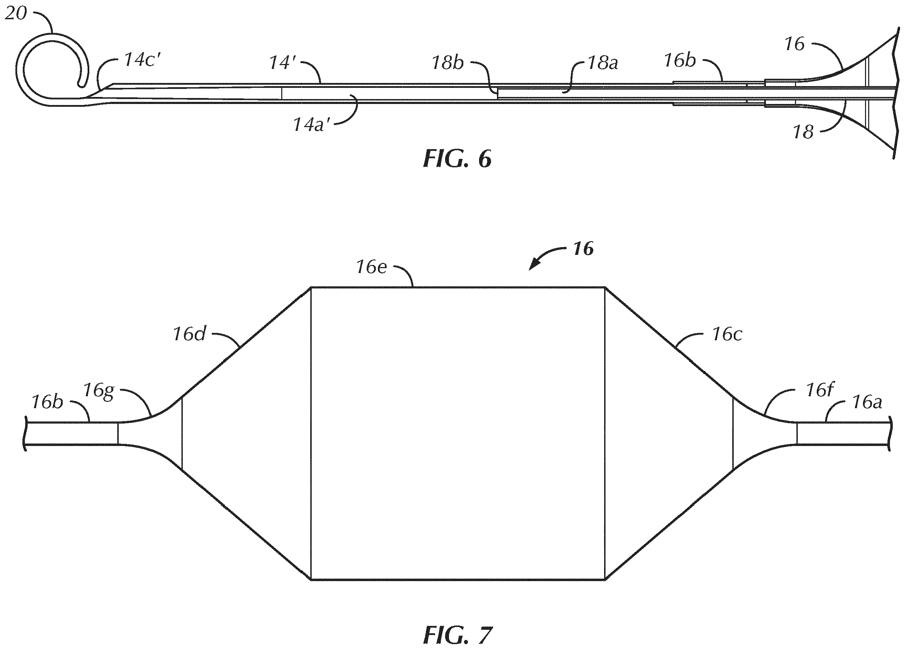

As shown best in Figs. 1 and 7, each of the respective neck portions 16a, 16b of the occlusion balloon 16 transitions into a respective, generally conical portion 16c, 16d (shown best in an inflated state), which together transition into a central body portion 16e. As shown in the illustrated configuration of Fig. 7, the proximal neck portion 16a and the proximal conical section 16c may define a generally radiused/curved transition section 16f therebetween. As also shown in the illustrated configuration of Fig. 7, the distal neck portion 16b and the distal conical section 16d may define a generally radiused/curved transition section 16g therebetween.

In one configuration, the occlusion balloon 16 may exhibit a large/oversized blown diameter and be constructed of a semi-compliant or substantially non-compliant material. In the context of the present disclosure, a non-compliant or substantially non-compliant balloon generally has growth of approximately two to seven percent (2-7%) within the working range (balloon pressure) when inflated, a semi-compliant balloon has growth of approximately seven to twenty percent (7-20%) within the working range (balloon pressure) when inflated and a compliant balloon has growth of approximately greater than twenty percent (20%+) within the working range (balloon pressure) when inflated. Compliant balloons, in contrast, may have growth of approximately one hundred to three hundred percent (100-300%) within the working range (balloon pressure) when inflated.

The occlusion balloon 16 may be "oversized", *i.e*., define a blown diameter D that is larger than a diameter of the target, *i.e*., destination, blood vessel (not shown) into which the occlusion balloon 16 is inserted and inflated for occlusion. In one configuration, the occlusion balloon 16 may define a blown diameter D between approximately twenty and approximately thirty-five millimeters (~20-35mm), which is configured to be between approximately ten percent and approximately sixty percent (10-60%) larger than the diameter of the target vessel, *e.g*., the aorta, in a normotensive state. The occlusion balloon 16 is, therefore, only partially inflated when its outer surface comes into full diametric contact/apposition with the inside wall of the target vessel and folds (not shown) remain at the peripheral surface of the occlusion balloon 16. The open/un-collapsed folds, in combination with the opposing inner wall of the vessel or with radially overlying portions of the outer surface of the occlusion balloon 16, result in flow channels (not shown) extending along the length of the occlusion balloon 16 that allow partial perfusion or blood flow past the occlusion balloon 16 under the blood pressure within the vessel, such as described, for example, in International Patent Application Publication No. WO 2022/197895 ("the `895 publication"), the entire contents of which are incorporated by reference herein in their entirety.

A central catheter shaft 18, *i.e.,* a hypotube, extends through the proximal catheter shaft 12, the occlusion balloon 16 and terminates within the distal catheter shaft 14 (as will be described in further detail below). The proximal catheter shaft 12 and the hypotube 18 may be secured/anchored at the respective proximal ends thereof to a proximal hub (not shown), e.g., to an internal frame thereof, such as, for example, as described in the `895 publication. The hypotube 18 forms the structural backbone/chassis of the catheter 10. Stated differently, the hypotube 18 forms the primary load-bearing framework of the catheter 10, advantageously eliminating the necessity for a separate guidewire to provide such load bearing stability. The hypotube 18 may be constructed of a metal, a metal alloy, a polymer, a combination thereof, *e.g*., composite braided shafts, or the like. Non-limiting examples of the hypotube 18 material include stainless steel, nitinol, plastic reinforced with fiberglass, Kevlar^{®} owned by E.I. Du Pont De Nemours and Company, and/or nylon. Generally, the hypotube 18 forms the most elastic shaft-component of the occlusion catheter 10, such that the hypotube 18 substantially returns to its initial configuration after a load is applied thereon and subsequently withdrawn. Optionally, the hypotube 18 may be constructed of a super-elastic material (e.g., such as nitinol). Alternatively, however, the hypotube 18, and at least one of the proximal catheter shaft 12 or the distal catheter shaft 14 may define substantially the same elasticity.

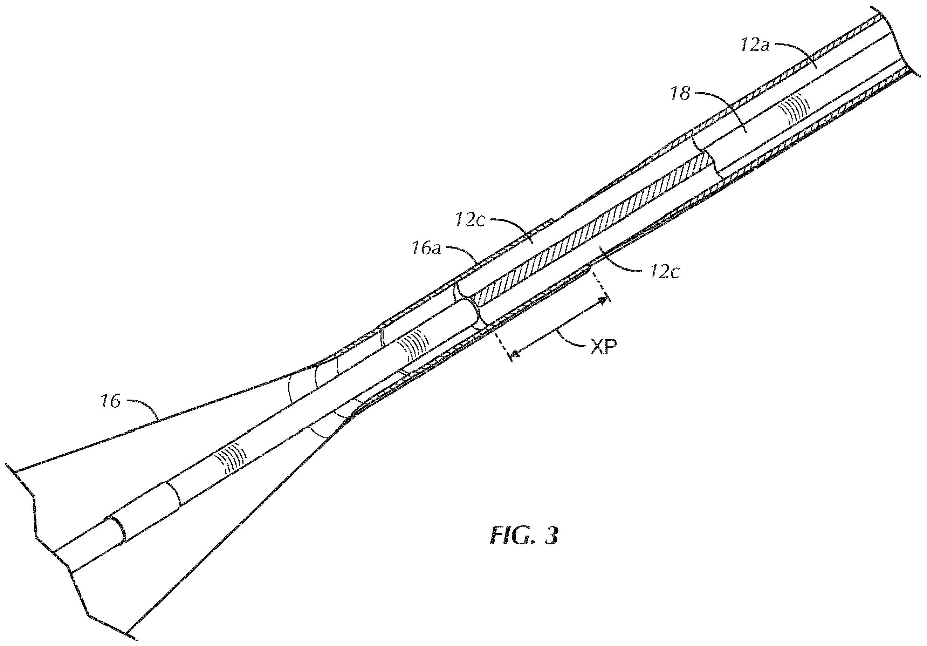

As shown best in Figs. 2 and 3, the hypotube 18 may define an inner hypotube lumen 18a. The proximal outer shaft 12 may include one or more discrete internal lumens (e.g., multiple, discretely extruded lumens). The hypotube 18 extends axially through a first internal lumen 12a of the proximal outer shaft 12, *i.e.,* the proximal shaft lumen 12a. Optionally, the proximal outer shaft 12 may also include a second internal lumen 12b, extending generally parallel with the proximal shaft lumen 12a along a portion of the extent thereof. A proximal sensor 22 may be included in the occlusion catheter 10, proximal to the occlusion balloon 16. In such a configuration, a proximal window 12d may be formed in the sidewall of the proximal catheter shaft 12 and extending into the second internal lumen 12b. The proximal sensor 22 may be positioned within the proximal window 12d, such as described, for example, in the `895 publication, and electrically connected with the proximal hub via the second internal lumen 12b.

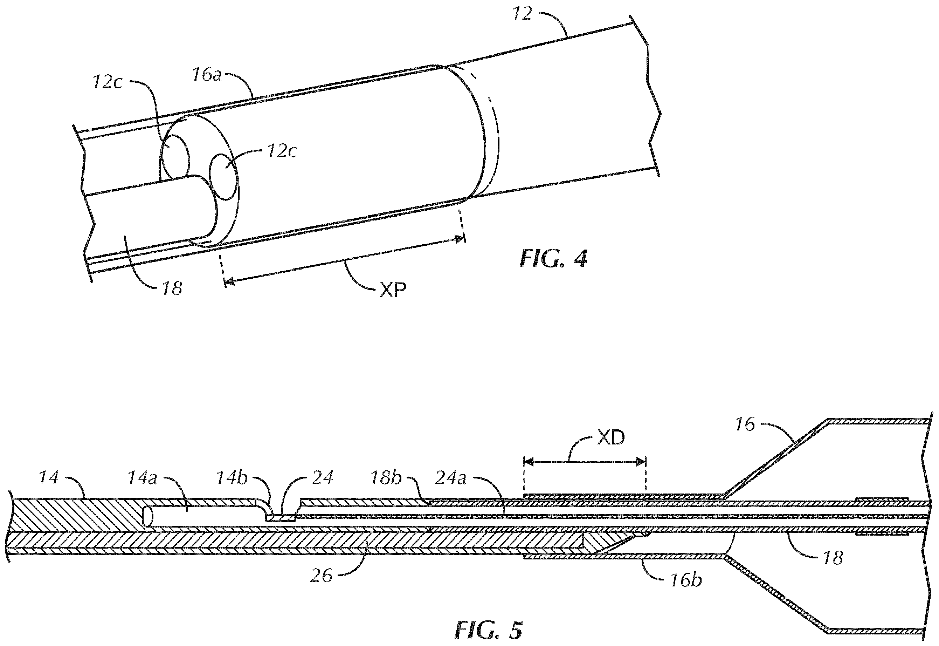

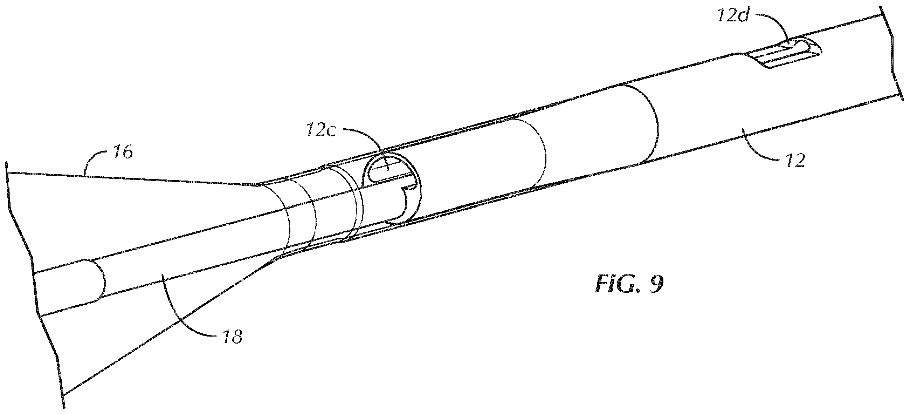

If employed, the second internal lumen 12b distally terminates proximally to the proximal neck portion 16a of the occlusion balloon 16. Distal to the second internal lumen 12b, the proximal catheter shaft 12 is adjoined to the hypotube 18. The proximal catheter shaft 12 may be adjoined and anchored to the hypotube 18 via welding, bonding (e.g., thermally bonded), a combination thereof or the like. As shown best in Figs. 3 and 4, the securement between the hypotube 18 and the proximal catheter shaft 12 may jacket the entire radial, e.g., circumferential, periphery of the hypotube 18, *i.e.,* a substantially 360° attachment, but the disclosure is not so limited. For example, as shown in Fig. 9, the hypotube 18 need only be partially, radially adjoined to the proximal catheter shaft 12, e.g., without limitation, between an approximately 120° attachment and an approximately 240° attachment. At least a segment of the proximal neck portion 16a is also adjoined (in like manner as aforementioned) to the proximal catheter shaft 12. The attachment between the proximal catheter shaft 12 and the hypotube 18 and the attachment between the proximal neck portion 16a and the proximal catheter shaft 12 at least partially, radially overlap along the axial axis of the catheter 10, thereby forming a proximal three-component-attachment XP (Fig. 4). The proximal three-component-attachment XP may define an axial length between approximately 0.5 mm and approximately 15 mm, such as, for example, between approximately 2 mm and approximately 10 mm.

The proximal shaft lumen 12a is in fluid communication with an inflation hub (not shown) on a proximal side thereof and is in fluid communication with the occlusion balloon 16 on a distal side. As shown best in Figs. 3 and 4, the proximal shaft lumen 12a transitions into at least one, internal transition lumen 12c extending through the bond between the proximal catheter shaft 12 and the hypotube 18. In the illustrated embodiment of Figs. 3 and 4, the proximal catheter shaft 12 includes two internal transition lumens 12c, but the disclosure is not so limited. For example, as shown in Fig. 9, only a single internal transition lumen 12c may be employed. Alternatively, more than two internal transition lumens 12c may be employed. Accordingly, inflation of the occlusion balloon 16 is provided via the free space of the proximal shaft lumen 12a unoccupied by the hypotube 18 and the internal transition lumen(s) 12c. If employed, the second internal lumen 12b is fluidly disconnected from the internal transition lumen(s) 12c.

One advantage of employing a single, larger internal transition lumen 12c, e.g., as shown in Fig. 9, relative to employing multiple, smaller internal transition lumens 12c, e.g., as shown in Figs. 3 and 4, is an overall reduction in friction, i.e., drag, counteracting the pressure required to produce a desired occlusion balloon fill rate. That is, the less catheter component surface area contacting the inflation fluid, the less drag counteracting the flow rate of the inflation fluid. Therefore, at a constant applied inflation fluid pressure, the inflation fluid will flow faster into the occlusion balloon 16 when flowing through a single, larger internal transition lumen 12c because of less drag as compared to when flowing through multiple, smaller internal transition lumens 12c. Another advantage of employing a single, larger internal transition lumen 12c over employing multiple, smaller internal transition lumens 12c is that, in view of the lower drag force within the single internal transition lumen 12c, the total cross-sectional area of the single internal transition lumen 12c may be smaller than the total combined cross-sectional area of the multiple internal transition lumens 12c and still provide the same occlusion balloon 16 fill rate. Such a reduction in the size of the single internal transition lumen 12c results in a narrowing of the proximal catheter shaft 12, which, in turn, results in a narrowing in the profile of the catheter 10. Such a reduction in the cross-sectional profile of the catheter 10 enables the formation of a catheter 10 that is less than 7 Fr, e.g., 6 Fr or 5 Fr. As should be understood by ordinary skill in the art, the narrower the catheter 10, the narrower the required introducer sheath, which, in turn, requires a smaller access site incision in the patient's body. Advantageously, the smaller the access site incision, the shorter amount of time is required to close the incision after completion of the procedure.

Turning to the distal side of the occlusion balloon 16 (Figs. 5, 6), and as previously described, the hypotube 18 extends through the occlusion balloon 16 and terminates in an open distal end 18b within the distal catheter shaft 14. The distal catheter shaft 14 includes a distal internal lumen 14a extending along at least a portion of the distal catheter shaft 14 from the proximal end thereof. The hypotube 18 extends into the distal internal lumen 14a, and, therefore, is substantially coaxial therewith. A distal portion of hypotube 18 is adjoined to the distal catheter shaft 14. The hypotube 18 may be adjoined and anchored to the distal catheter shaft 14 via welding, bonding (e.g., thermally bonded), a combination thereof or the like. At least a segment of the distal neck portion 16b of the occlusion balloon 16 is also adjoined (in like manner as aforementioned) to distal catheter shaft 14. The attachment between the distal catheter shaft 14 and the hypotube 18 and the attachment between the distal neck portion 16b and the distal catheter shaft 14 at least partially, radially overlap along the axial axis of the catheter 10, thereby forming a distal three-component-attachment XD (Fig. 5). The distal three-component-attachment XD may define an axial length between approximately 0.5 mm and approximately 20 mm, such as, for example, between approximately 2 mm and approximately 10 mm.

The proximal three-component-attachment XP effectively adjoins, e.g., bonds, the proximal neck portion 16a of the occlusion balloon 16 to the hypotube 18, and the distal three-component-attachment XD effectively adjoins, e.g., bonds, the distal neck portion 16b of the occlusion balloon 16 to the hypotube 18. As previously described, the occlusion balloon 16 is mounted to the catheter 10 with a requisite amount of the axial slack removed, such that the uninflated/folded balloon 16 is also seven French (7 Fr) or smaller in radial cross-section. Consequently, upon inflation of the at least partially taut/stretched occlusion balloon 16, *i.e*., increasing balloon diameter, the distance between the proximal and distal neck portions 16a, 16b decreases. In doing so, the proximal and distal neck portions 16a, 16b create oppositely directed forces toward the body 16e of balloon 16, which translate into separate tensile forces onto each of the proximal and distal catheter shafts 12, 14, directed toward the body 16e of the occlusion balloon 16.

Advantageously, the proximal and distal three-component-attachments XP, XD relieve the oppositely directed forces from the proximal and distal catheter shafts 12, 14, by transferring the forces onto the hypotube 18, which is the structural chassis of the catheter 10 and the most elastic shaft-component of the catheter 10. The oppositely directed forces on the hypotube 18 form a compressive force on the hypotube 18 within the occlusion balloon 16. The hypotube 18 is configured, e.g., via the material properties thereof, to elastically bend/bow in response, thereby accommodating the decrease in distance between proximal and distal neck portions 16a, 16b of the inflated balloon 16 while alleviating the tensile forces on the proximal and distal catheter shafts 12, 14.

Upon subsequent deflation of the occlusion balloon 16, stored elastic energy in the elastically bowed hypotube 18 substantially straightens the hypotube 18 back out and re-stretches the occlusion balloon 16 in the deflated state to substantially the same state as prior to inflation. Advantageously, therefore, the outer radial profile of the catheter 10 remains substantially the same in the pre-inflation, uninflated state as in the post-inflation, deflated state, and, in turn, withdrawal of the catheter 10 from the introducer sheath (not shown) is unaffected. For example, the catheter 10 may be dimensioned to pass through a 7 Fr introducer sheath at the outset of the REBOA procedure and return to substantially the same dimensions (or at least sufficient dimensions) to withdraw in like manner, *i.e*., without increased difficulty, from the 7 Fr introducer sheath upon completion of the REBOA procedure.

Turning again to the distal side of the occlusion balloon 16, as shown in Fig. 5, a distal sensor 24 may, in one configuration, also be employed in the occlusion catheter 10, distal to the occlusion balloon 16. In such a configuration, a distal window 14b is formed in the sidewall of the distal catheter shaft 14 and extends into the distal internal lumen 14a. The distal sensor 24 is positioned within an internal distal lumen 14a facing the distal window 14b and a distal sensor signal wire 24a may extend proximally from the distal sensor 24 along a portion of the internal distal lumen 14a and through the hypotube 18 to the proximal hub, such as described, for example, in the `895 publication.

In one configuration, as also shown in Fig. 5, a solid distal wire 26 may be embedded in the distal catheter shaft 14, adjacent and generally parallel to the hypotube 18 and the distal internal lumen 14a. The solid distal wire 26 may be constructed of the same material as the hypotube 18 or may be constructed of another one of the suitable hypotube materials previously identified. Such materials, such as, for example, without limitation, nitinol, have super-elastic properties providing advantageous kink resistance. A proximal portion of the distal wire 26 may overlap with a distal portion of the hypotube 18 and a distal portion of the internal distal lumen 14a. Optionally, at least a section of the proximal portion of the distal wire 26 overlapping the distal portion of the hypotube 18 may be jacketed to the hypotube 18 by a polymeric sleeve or by the distal outer shaft 14 itself. The distal wire 26 may alternatively be welded or otherwise bonded to the hypotube 18, *i.e.,* affixed without relying on a jacket. The distal wire 26 may extend toward a distal end of the catheter 10 and may taper from a proximal end thereof to a distal end thereof, thereby progressively reducing stiffness of the catheter 10 from the proximal end of the distal outer shaft 18 to the distal end thereof.

In an alternative configuration, not employing the solid distal wire 26 (as shown in Fig. 6), the distal internal lumen 14a' may extend through the entirety of the distal catheter shaft 14' to an open distal port 14c' at the base of the atraumatic tip 20. The hypotube 18, being in fluid communication with the distal internal lumen 14a', therefore, fluidly communicates the distal port 14c' with the proximal hub. In the configuration of Fig. 6, the distal sensor 24 is optional.

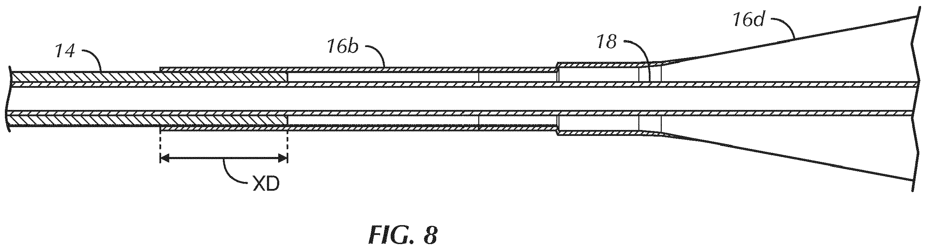

Turning more specifically to the occlusion balloon 16, in one configuration, and as previously described, the entirety of the balloon 16 may be constructed of the same material, e.g., a polymeric semi-compliant or substantially non-compliant material. In an alternative configuration, the occlusion balloon 16 may be constructed of multiple polymeric materials. For example, as shown in Figs. 7 and 8, the central body portion 16e and the generally conical portions 16c, 16d may be constructed of a semi-compliant or substantially non-compliant polymeric material, e.g., higher durometer Pebax, whereas at least one or both of the proximal and distal neck portions 16a, 16b (or sections thereof) may be constructed of a relatively lower durometer polymeric material, e.g., lower durometer Pebax, having, for example, without limitation, between approximately 0.5% and approximately 80% of the tensile modulus of the material of the central body portion 16e. Employing lower durometer neck portion(s) of the occlusion balloon 16 provides an elastic, spring-like effect to the axial dimension of the occlusion balloon 16, wherein the balloon 16 remains sufficiently stiff to be assembled to the catheter in an at least partially tensioned/taut pre-inflation, uninflated state. As the balloon 16 is inflated, however, the lower durometer neck portion(s) 16a and/or16b may elastically elongate and thereafter elastically return to substantially the same degree of tension/tautness in the post-inflation, deflated state, for uncomplicated insertion/removal through the introducer sheath. Advantageously, constructing one or both of the balloon of neck portions 16a, 16b of lower durometer polymers relative to the central body portion 16e mitigates bunching of the balloon material at the transition sections between the generally conical portions 16c, 16d and the corresponding neck portions 16a, 16b, which may otherwise increase the radial profile of the balloon in the post-inflation, deflated state.

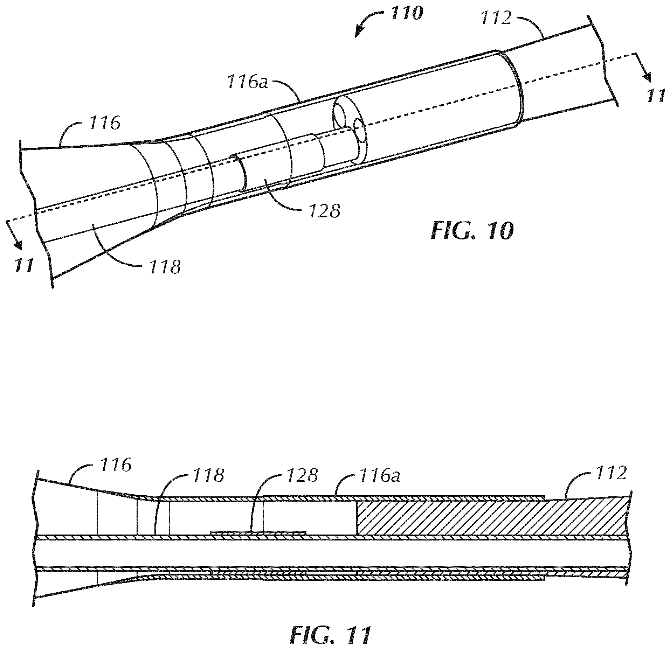

Figs. 10-13 illustrate a second embodiment of the occlusion catheter 110. The reference numerals of the second embodiment are distinguishable from those of the above-described embodiment by a factor of one-hundred (100), but otherwise indicate the same or similar elements as indicated above, except as otherwise specified. The occlusion catheter 110 of the present embodiment is similar to that of the earlier embodiment. Therefore, the description of certain similarities between the embodiments may be omitted herein for the sake of brevity and convenience, and, therefore, is not limiting.

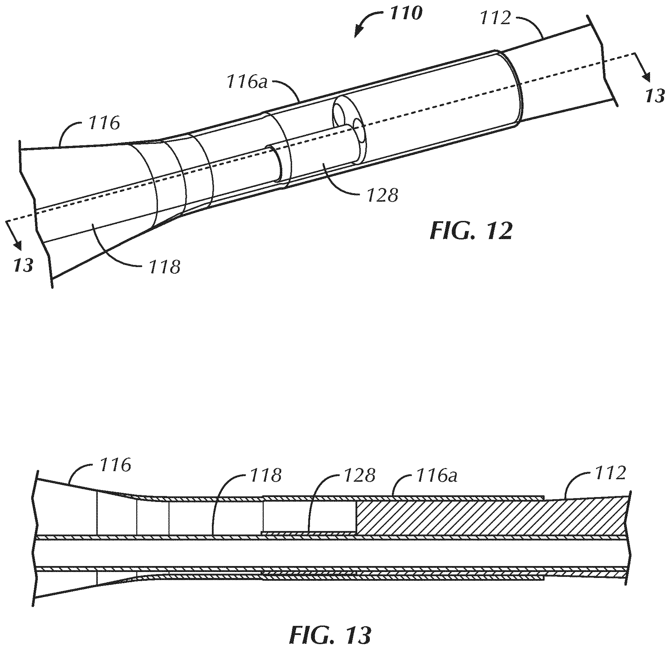

In the occlusion catheter 110 of the second embodiment, a ring 128 is adjoined, e.g., bonded, to the hypotube 118. The ring 128 is positioned distally of the distal end of the proximal catheter shaft 112, within the proximal neck portion 116a of the occlusion balloon 116. Unlike the occlusion catheter 10, the distal end of the proximal catheter shaft 112 is not adjoined to the hypotube 118. The ring 128 is sufficiently distally spaced (a predetermined distance) from the proximal catheter shaft 112 (Figs. 10 and 11) to permit elastic elongation of the proximal catheter shaft 112 while also substantially protecting against additional plastic axial deformation of the proximal catheter shaft 112. That is, when the occlusion balloon 116 is inflated during use, whether partially or fully, the occlusion balloon 116 applies a tensile force on both opposing neck portions of the balloon 116 while the central diameter of the occlusion balloon 116 increases. In turn, the neck portions of the occlusion balloon 116 transfer the tensile forces onto other components of the catheter 110. For example, the proximal neck portion 116a applies a tensile force onto the proximal catheter shaft 112 (via the bond therebetween). As the proximal catheter shaft 112 is not adjoined to the hypotube 118, the force is not further transferred from the proximal catheter shaft 112 to the hypotube 118 (as in the catheter 10), but rather the tensile force elongates the proximal catheter shaft 112. As shown in Figs. 12 and 13, the ring 128 is positioned to allow the proximal catheter shaft 112 to elongate substantially within the elastic region thereof and operates as a stop surface to substantially prevent the proximal shaft 112 from reaching plastic axial deformation. Therefore, upon subsequent deflation of the occlusion balloon 116, stored elastic energy in the proximal catheter shaft 112 returns the proximal catheter shaft 112 substantially to the original length thereof.

It will be appreciated by those skilled in the art that changes could be made to the embodiment(s) described above without departing from the broad inventive concept thereof. It is understood, therefore, that this disclosure is not limited to the particular embodiments disclosed, but it is intended to cover modifications within the spirit and scope of the present disclosure as defined by the present description, as set forth in the appended claims.

## Claims

1. A vascular occlusion catheter (10) for at least partial occlusion of a target vessel, the vascular occlusion catheter (10) comprising:
a proximal catheter shaft (12) having at least one internal lumen (12a, 12b, 12c);
a distal catheter shaft (14, 14') terminating in an atraumatic tip (20);
an occlusion balloon (16) sealingly mounted to the proximal and distal catheter shafts (12;14, 14'); and
a central catheter shaft (18) extending through the proximal catheter shaft (12), the occlusion balloon (16) and into the distal catheter shaft (14, 14'), the proximal catheter shaft (12) being secured to the central catheter shaft (18) on a proximal side of the occlusion balloon (16) and the distal catheter shaft (14, 14') being secured to the central catheter shaft (18) on a distal side of the occlusion balloon (16),
wherein the occlusion balloon (16), the proximal catheter shaft (12) and the distal catheter shaft (14, 14') have a greatest outer diameter of less than seven French (7 Fr) in an uninflated condition.

2. The vascular occlusion catheter (10) of claim 1, wherein a proximal neck portion (16a) of the occlusion balloon (16) is thermally bonded to the proximal catheter shaft (12) and a distal neck portion (16b) of the occlusion balloon (16) is thermally bonded to the distal catheter shaft (14, 14'), and wherein each of the proximal and distal neck portions (16a, 16b) transitions into a generally conical portion (16c, 16d).

3. The vascular occlusion catheter (10) of claim 2, wherein at least one of (i) the proximal neck portion (16a) and the corresponding generally conical portion (16c) define a radiused transition (16f) therebetween, or (ii) the distal neck portion (16b) and the corresponding generally conical portion (16d) define a radiused transition (16g) therebetween.

4. The vascular occlusion catheter (10) of any one of the previous claims, wherein the central catheter shaft (18) extends through a first internal lumen (12a) of the at least one internal lumen (12a, 12b, 12c) of the proximal catheter shaft (12) and the at least one internal lumen (12a, 12b, 12c) further includes a second internal lumen (12b).

5. The vascular occlusion catheter (10) of claim 4, further comprising a proximal window (12d) formed in a sidewall of the proximal catheter shaft (12) and extending into the second internal lumen (12b), a proximal sensor (22) positioned within the proximal window (12d).

6. The vascular occlusion catheter (10) of claim 4, wherein the second internal lumen (12b) distally terminates proximal to the occlusion balloon (16).

7. The vascular occlusion catheter (10) of claim 6, wherein the proximal catheter shaft (12) is thermally bonded to the central catheter shaft (18) distal to the second internal lumen (12b).

8. The vascular occlusion catheter (10) of any one of the previous claims, wherein the proximal catheter shaft (12) is thermally bonded to the central catheter shaft (18) and at least a segment of a proximal neck portion (16a) of the occlusion balloon (16) is thermally bonded to the proximal catheter shaft (12), the bond between the proximal neck portion (16a) and the proximal catheter shaft (12) at least partially radially overlapping with the bond between the proximal catheter shaft (12) and the central catheter shaft (18), along an axial axis of the catheter (10), thereby forming a proximal three-component-attachment (XP).

9. The vascular occlusion catheter (10) of any one of the previous claims, wherein the proximal catheter shaft (12) is thermally bonded to the central catheter shaft (18) and the at least one internal lumen (12a, 12b, 12c) includes a proximal shaft lumen (12a) which distally transitions into at least one internal transition lumen (12c) axially extending entirely through the thermal bond, thereby fluidly connecting the proximal shaft lumen (12a) with an interior of the occlusion balloon (16).

10. The vascular occlusion catheter (10) of any one of the previous claims, wherein the securement between the proximal catheter shaft (12) and the central catheter shaft (18) jackets an entire circumferential periphery of the central catheter shaft (18).

11. The vascular occlusion catheter (10) of any one of the previous claims, wherein the distal catheter shaft (14, 14') is thermally bonded to the central catheter shaft (18) and at least a segment of a distal neck portion (16b) of the occlusion balloon (16) is thermally bonded to the distal catheter shaft (14, 14'), the bond between the distal neck portion (16b) and the distal catheter shaft (14, 14') at least partially radially overlapping with the bond between the distal catheter shaft (14, 14') and the central catheter shaft (18), along an axial axis of the catheter (10), thereby forming a distal three-component-attachment (XD).

12. The vascular occlusion catheter (10) of any one of the previous claims, wherein the central catheter shaft (18) extends into a distal internal lumen (14a) of the distal catheter shaft (14), a distal window (14b) being formed in a sidewall of the distal catheter shaft (14) and extending into the distal internal lumen (14a), and further comprising a distal sensor (24) positioned within the distal window (14b).

13. The vascular occlusion catheter (10) of any one of the previous claims, wherein the distal catheter shaft (14') defines a distal internal lumen (14a') terminating in an open distal port (14c') proximate the atraumatic tip (20), the central catheter shaft (18) distally terminating within the distal internal lumen (14a')and being in fluid communication with the open distal port (14c').

14. The vascular occlusion catheter (10) of any one of the previous claims, wherein the occlusion balloon (16) defines a proximal neck portion (16a), a distal neck portion (16b) and an intervening central body portion (16e), wherein at least one of the proximal neck portion (16a) or the distal neck portion (16b) is constructed of a material having a lower durometer than the central body portion (16e).

15. The vascular occlusion catheter (10) of claim 14, wherein the at least one of the proximal neck portion (16a) or the distal neck portion (16b) defines a tensile modulus that is between approximately 0.5% and approximately 80% of a tensile modulus of the central body portion (16e).
